# EUROPÄISCHE PATENTANMELDUNG

(11) **EP 2 005 970 A1**
(43) Veröffentlichungstag der Anmeldung: **24.12.2008**
(21) Anmeldenummer: 07110922.7
(22) Anmeldetag: 22.06.2007
(51) Int. Cl.: A61K 49/04, A61K 51/04, A61K 49/06

(54) **Bildgebende Diagnostik durch Kombination von Kontrastmitteln**

(71) Anmelder: Berlin Science Partners GmbH i.V., 14089 Berlin (DE)
(72) Erfinder: Wiebelitz, Ulrike, 13158 Berlin (DE)
(74) Vertreter: Vossius & Partner

(57) **Zusammenfassung**

Die vorliegende Erfindung betrifft die Verwendung einer Kombination mehrerer Kontrastmittel mit unterschiedlichen Eigenschaften für die bildgebende Darstellung.

## Beschreibung

### Einleitung

Die vorliegende Erfindung betrifft die Verwendung einer Kombination mehrerer Kontrastmittel mit unterschiedlichen Eigenschaften für die bildgebende Darstellung.

### Stand der Technik

Chronische Erkrankungen stellen ein großes Anwendungsfeld für die bildgebende Diagnostik dar. Alleine die zwei häufigsten chronischen Erkrankungen, die Herz-Kreislauferkrankungen und die Tumorerkrankungen bedingen einen großen Teil der jährlich weltweit 800 Mio. bildgebenden Untersuchungen. Die Mehrzahl der Untersuchungen sind Ultraschalluntersuchungen, Untersuchungen mittels Röntgenstrahlen, wie CT und MRT-Untersuchungen. Aber auch nuklearmedizinische und optische Verfahren werden häufig angewendet. Für alle diese Untersuchungsverfahren sind Kontrastmittel klinisch verfügbar. Kontrastmittel liefern ganz bestimmte Aussagen, die durch ihre pharmakokinetischen und pharmakodynamischen Eigenschaften bestimmt sind. Durch eine bildgebende Untersuchung allein werden aber sehr oft nur wenige diagnostische Informationen erarbeitet. Aus diesem Grund müssen sehr häufig verschiedene Untersuchungen mit derselben Modalität und unterschiedlichen Kontrastmitteln oder verschiedene Untersuchungen mit unterschiedlichen Modalitäten durchgeführt werden, um eine verlässliche Diagnose zu ermöglichen. Dieses Vorgehen ist mit hohen Kosten und Belastungen für den Patienten verbunden. Darüber hinaus ist dieses Vorgehen langwierig und oft wird eine notwendige Therapie wegen der Notwendigkeit, mehrere diagnostische Untersuchungen durchzuführen, erst sehr verzögert eingeleitet. Ein weiterer Nachteil ist die Zuordnung diagnostischer Signale aus verschiedenen bildgebenden Untersuchungen. Sehr oft können verdächtige Läsionen in einer Untersuchung nicht den Läsionen einer anderen Untersuchung zugeordnet werden. Hierdurch wird dem Arzt die Diagnose deutlich erschwert. Die Notwendigkeit, verschiedene diagnostische Untersuchungen durchzuführen, um eine verlässliche Diagnose zu ermöglichen, kann am Beispiel der bildgebenden Diagnostik von Tumoren, speziell der bildgebenden Diagnostik von Mammatumoren, sehr eindrucksvoll verdeutlicht werden.

Das Mammakarzinom ist eine der häufigsten Tumore und die häufigste Tumorerkrankung bei der Frau. Durch eine verbesserte Frühdiagnostik, wie z.B. die Screening-Mammographie und komplexe Behandlungsprotokolle konnte die Sterblichkeit in den letzten Jahren gesenkt werden. Trotzdem wird jährlich eine Vielzahl von Mammatumoren erst in einem späten Stadium entdeckt. Aus diesem Grund ist die größte Herausforderung die rechtzeitige Diagnostik der Tumoren. Dies ist besonderes wichtig, da das Wachstum kleiner Tumoren auf das Organ begrenzt ist und somit die Hoffnung besteht, durch rechtzeitige Entdeckung der frühen Tumorstadien die Tumoren vollständig und organerhaltend entfernen zu können. Die bisher verfügbaren medizinischen Verfahren erfüllen diesen Anspruch nur unzureichend. Deshalb ist es wichtig, verbesserte Verfahren zur Früherkennung und sicheren Diagnose zur Verfügung haben.

Der Arzt kann heute auf verschiedene diagnostische Verfahren zurückgreifen, wobei bildgebende Verfahren die wichtigste Rolle spielen. Unter den bildgebenden Verfahren kommt der Ultraschalldiagnostik, der CT, der MRT und den nuklearmedizinischen Verfahren, wie PET und SPECT, die größte Bedeutung zu. Bei unklarem Befund ist es das Ziel, Gewebe aus den verdächtigen Läsionen zu entnehmen und histopathologisch aufzuarbeiten und zu bewerten.

Die MRT-Untersuchung der weiblichen Brust hat im Vergleich zu anderen bildgebenden Modalitäten eine sehr hohe Sensitivität. Durch diese Modalität können verschiedene Formen von Mammatumoren in frühen Stadien nachgewiesen werden. Dabei ist es eine besondere Stärke der MRT, dass nicht-tumoröse Gewebeveränderungen, wie z.B. Operationsnarben, Gewebeveränderungen durch Strahlenbehandlungen, Prothesen oder mastopathisch verändertes Drüsengewebe die bildgebende Untersuchung nicht relevant erschweren. Aufgrund dieser Eigenschaften hat sich zwischenzeitlich ein breites Anwendungsspektrum für die MRT etabliert.

Die hohe Empfindlichkeit der MRT an der Mamma (Brust) ist jedoch mit einer niedrigen Spezifität verbunden. Eine geringe Spezifität bedeutet, dass zwar fast alle bösartigen Tumoren entdeckt werden, aber auch sehr viele Herde als bösartige Tumoren imponieren, die sich im Verlauf weiterer Untersuchungen als harmlos darstellen. Dies ist darin begründet, dass bei der MRT Kontrastmittel verwendet werden, die das Verfahren soweit verbessern, dass bereits geringe Unregelmäßigkeiten im zu untersuchenden Brustgewebe dargestellt werden können. Diese Kontrastmittel können aber nur sehr unzureichend zwischen gutartigen und bösartigen Läsionen unterscheiden. In der Konsequenz dieser Ergebnisse ist der Arzt gezwungen, diagnostische Folgeuntersuchungen einzuleiten, die die Dignität der verdächtigen Befunde genauer bestimmen sollen. Zum einen werden weitere bildgebende Verfahren angewendet, zum anderen besteht die Möglichkeit, eine MRT-gestützte Biopsie des verdächtigen Gewebes durchzuführen und durch die Gewebeuntersuchung eine genaue Diagnose zu erstellen. Dieses Verfahren ist technisch anspruchsvoll und mit hohen Kosten verbunden. Dies ist einer der Gründe, warum die MRT Bildgebung noch nicht zu einem Standardverfahren bei der Mammatumordiagnostik geworden ist. Eine vergleichbare Situation ist bei der klinischen Anwendung der CT festzustellen. Die CT spielt bei der Tumordiagnostik ebenfalls eine wichtige Rolle. So wird z.B. das CT-Kontrastmittlel Ultravist zur bildgebenden CT-Diagnostik von Lebertumoren eingesetzt. Auch bei diesem Verfahren ist die Spezifität des Kontrastmittelbefunds gering. Es ist somit eine zentrale Aufgabe, die Spezifität der diagnostischen Bildgebung zu verbessern, um ihr eine breite Anwendung zu ermöglichen.

Eine Möglichkeit die Aussagekraft einer bildgebenden Modalität zu verbessern, ist die Anwendung verbesserter signalverändernde oder signalmodulierende Kontrastmittel. Für die meisten bildgebenden Verfahren sind heute Kontrastmittel klinisch verfügbar. Diese Kontrastmittel wurden so ausgewählt, dass sie einerseits gut verträglich am Menschen angewendet werden können und andererseits sehr spezifisch mit dem physikalischen Signal der jeweiligen Untersuchungsmodalität interferieren können. Die meisten Erfahrungen zur Anwendung von Kontrastmitteln liegen bei den Röntgenstahlen-basierten CT-Untersuchungsverfahren vor. Bei der Röntgenuntersuchung werden Kontrastmittel angewendet, welche die Röntgenstrahlen abschwächen. Das sind u.a. Wirkstoffe mit einer hohen Zahl an elektronendichten Elementen, wie z.B. Jod. Diese Kontrastmittel können auf unterschiedliche Weise am Menschen angewendet werden, jedoch sind die Anwendungen, bei denen die Kontrastmittel in einer Bolusinjektion in die Blutzirkulation eingebracht werden, am häufigsten. Hierdurch wird für die Zeit der Untersuchung der Blutfluss in einem bestimmten Organsystem sichtbar gemacht. Werden Kontrastmittel in dieser Weise angewendet, können wichtige Informationen zur Anatomie, Morphologie und Funktion bestimmte Organe erhalten werden. So lassen sich z.B. durch den Nachweis eines Kontrastmittels im Blutgefäß und durch die Darstellung der Gefäßlumen Aussagen über den Zustand des Blutgefäßes gewinnen. Liegen Gefäßverengungen der Koronararterien vor, können diese im Rahmen einer Koronar-Angiographie ohne Schwierigkeiten dargestellt werden und begründen somit die Diagnose einer Koronarstenose. Ein weiterer Parameter, der wichtige diagnostische Informationen liefert, ist die Geschwindigkeit, mit der das applizierte Kontrastmittel in ein Organ einfließt. Hierdurch können Rückschlüsse auf die Durchblutung eines Organs gezogen werden.

Die Anwendung von Kontrastmitteln spielt bei der MRT eine ebenso große Rolle wie bei den Röntgenuntersuchungen. Sowohl für die Röntgenstrahlen-basierten Untersuchungen als auch die MRT-Untersuchungen steht dem Arzt eine Vielzahl verschiedener Kontrastmittel zur Verfügung. Alle diese Kontrastmittel sind jedoch für bestimmte Verwendungszwecke optimiert. Für die Detektion von Gewebeläsionen, wie z.B. Tumoren, Erkrankungen des ZNS und Erkrankungen des Herz-Kreislaufsystems spielen die extrazellulären Kontrastmittel (ECCM; extracellular contrast media) eine zentrale Rolle. Diese Kontrastmittel sind so optimiert, dass sie nach der Applikation von einer sehr hohen Maximalkonzentration im Blut sehr schnell in wenigen Minuten auf ein sehr niedriges Konzentrationsniveau im Blut zurückgehen (alpha slope der Blutkinetik). Sie sind darüber hinaus durch eine Plasmaproteinbindung von weniger als 90%, vorzugsweise weniger als 70% charakterisiert. Aufgrund ihrer kleinen Molekülgröße und unvollständigen Bindung an Plasmaproteine können sie sehr leicht die Kapillarschranke überwinden. Eine weitere wichtige Eigenschaft von ECCM ist die fehlende Interaktion mit biologischen Stukturen. ECCM binden nicht an bestimmte Strukturen in der zu untersuchenden Läsion, bzw. werden nicht von diesen Strukturen verändert. Durch diese Eigenschaften können sie die Kapillarschranken in beiden Richtungen gut passieren und dadurch verdächtige Läsionen im Vergleich zum umliegenden gesunden Gewebe gut demarkieren. Dieser Prozess wird als Extravasation bezeichnet und findet auch bei einem intakten Kapillarbett statt, aber mit deutlich geringerer Geschwindigkeit. Vor allem bei Tumorerkrankungen, Erkrankungen des ZNS und Erkrankungen des Herz-Kreislaufsystems ist die Kapillarschranke defekt und die ECCM treten an diesen Stellen bevorzugt aus. Die Extravasation der ECCM führt in der Läsion gegenüber der gesunden Umgebung zu einer Signalerhöhung. Innerhalb der ersten Minuten nach Injektion eines ECCM können Signalerhöhungen bis zu 100 % festgestellt werden. Diese erste Phase des Extravasationsprozesses wird auch als wash-in bezeichnet. An diese Phase kann sich eine schnelle, oft jedoch aber inkomplette Auswaschphase anschließen. Das Signal in den Läsionen kann auch geringer als in der Umgebung sein, da der Auswaschprozess in der Läsion aufgrund der gestörten Schrankenfunktionen schneller stattfinden kann als im gesunden Gewebe, da hier die intakte Schranke die Rücktransportgeschwindigkeit vermindert. Die treibende Kraft für den Rücktransport ist jedoch immer die schnelle Abnahme der Blutkonzentration des ECCM. Diese Eigenschaft charakterisiert die erfindungsgemäß verwendeten ECCM. Eine schnelle Elimination aus der Blutzirkulation setzt eine unvollständige und lockere Plasmaproteinbindung voraus und wird durch eine renale und / oder hepatische Eliminationsfähigkeit gewährleistet. Im Unterschied zu den ECCM zeichnen sich die erfindungsgemäß verwendeten läsionsspezifischen Kontrastmittel (LSCM; lesion-specific contrast media) dadurch aus, dass sie mit einer bestimmten Zielstruktur im Organismus in Wechselwirkung treten können oder durch diese bestimmten Zielstrukturen in der Weise verändert werden, dass sie als Folge der Wechselwirkung oder Veränderung bildgebend dargestellt werden können. Eine weitere Eigenschaft, durch die sie sich von den ECCM unterscheiden können, ist das längere Verweilen in der Blutzirkulation. Diese Eigenschaft kann z.B. dadurch erreicht werden, dass die betreffenden LSCM fester durch eine Plasmaproteinbindung von mehr als 90% charakterisiert sind.

Das wichtigste ECCM für die MRT ist Gadolinium-DTPA (Gd-DTPA). Gd-DTPA ist eine paramagnetische Substanz, die zu einer Verkürzung der T1-Relaxationszeit des umgebenden Gewebes führt. Es ist eine Substanz mit kleinem Molekulargewicht, tritt nicht in Wechselwirkungen mit Strukturen des Organismus und wird durch diese nicht verändert. Durch diese Eigenschaften ist es in der Lage, das Gefäßbett auch bei intakter Kapillarschranke und normaler Gefäßpermeabilität rasch zu verlassen. Gd-DTPA, welches das Gefäßbett verlassen hat, verteilt sich im Extravasalraum. Viele Erkrankungen, wie z.B. Tumorerkrankungen, entzündliche Organveränderungen oder Gewebeschäden beim Schlaganfall führen zu einer Schädigung der Kapillarschranke. Werden bei Patienten mit solchen Erkrankungen MRT Untersuchungen mit dem Kontrastmittel Gd-DTPA durchgeführt, tritt dieses Kontrastmittel verstärkt in Bereichen mit gestörter Kapillarschranke aus. Speziell in diesen Bereichen kommt es zur Erhöhung der Konzentrationen im Gewebe, die im MRT-Bild durch ein stärkeres Signal auffällt. Diesem Effekt folgt die verstärkte Rückdiffusion, die auch als Auswaschphänomen bezeichnet wird. Dieses charakteristische Verhalten, das auch als "wash-in / wash-out"-Phänomen bezeichnet wird, kann bei der MRT Diagnostik vielfältig genutzt werden, wie z.B. bei der Detektion von Tumoren mittels schneller Scan-Zeiten direkt während der Kontrastmittelinjektion im MRT. Speziell bei der bildgebenden Diagnostik von verdächtigen Gewebeveränderungen an der weiblichen Brust hat sich gezeigt, dass durch das "wash-in / wash-out"-Phänomen sicher alle bösartigen Läsionen ab einer bestimmten Größe in der Brust entdeckt werden können. Dieses Verfahren hat jedoch auch einen großen Nachteil. Zusätzlich zu den tatsächlich vorhandenen malignen Tumoren werden auch viele Herde entdeckt, die gutartig sind. Es handelt sich hierbei um eine Vielzahl verschiedener gutartiger Gewebeveränderungen, von denen keine Gefahr für die Patienten ausgeht. Bisher ist kein Verfahren bekannt, welches die Spezifität von sehr empfindlichen bildgebenden Methoden in befriedigender Weise erhöht.

Ein möglicher Weg, die Spezifität bildgebender Untersuchungsverfahren zu verbessern, ist die Durchführung verschiedener kontrastmittelgestützter Untersuchungen. Aus der Literatur sind Verfahren bekannt, bei denen die verdächtigen Krankheitsläsionen nacheinander mit zwei verschiedenen MRT-Kontrastmitteln charaktarisiert wurden. Bei diesem monomodalen Verfahren wurde die Untersuchung in zwei Abschnitten durchgeführt. Zuerst wurde ein LSCM angewendet. Durch die lange Verweildauer konnte erst durch eine zeitlich abgesetzte zweite Untersuchung die Anreicherung eines ECCM untersucht werden. Dieses Vorgehen hat den entscheidenden Nachteil, dass durch die getrennt durchgeführten Untersuchungen eine Überlagerung beider Signale nur eingeschränkt möglich ist. Dadurch verliert das in der Literatur bekannte Verfahren an Sensitivität und Spezifität und bietet keinen Vorteil [Marcarini L et.al; Radiol Med (2006) 111: 1087-1102].

### Beschreibung der Erfindung

Überraschenderweise wurde gefunden, dass die Verwendung von ECCM in Kombination mit mindestens einem weiteren bildgebenden Kontrastmittel oder signalgebenden Wirkstoffs, der sich spezifisch in der Krankheitsläsion anreichern kann (läsionspezifische Kontrastmittel / LSCM) ein völlig neues bildgebendes Verfahren darstellt, das eine deutlich höhere Spezifität aufweist als die alleinige Anwendung von ECCM. Die erfindungsgemäße Kombination ist dadurch charakterisiert, dass das ECCM und das LSCM zum gleichen Zeitpunkt angewendet werden, oder das LSCM zeitlich versetzt in einem kurzen Zeitabstand von maximal 30 Minuten, bevorzugt maximal 20 Minuten, am meisten bevorzugt maximal 10 Minuten nach Applikation des ECCM angewendet wird. Das erfinderische Verfahren gestattet somit die Interpolation der unterschiedlichen bildgebenden Signale und erreicht eine Läsionsdiagnostik mit hoher Sensitivität und hoher Spezifität. Interpolation bedeutet, dass in einem räumlich unveränderten Untersuchungsfeld /-raum mindestens zwei voneinander verschiedene Signale räumlich und zeitlich miteinander in Beziehung gesetzt werden (können). Nachteile des Standes der Technik, bei denen also vollkommen getrennte Diagnoseverfahren durchgeführt werden, werden also erfindungsgemäß dadurch überwunden, dass die Kombination von ECCM und LSCM eine sinnvolle Interpolation der unterschiedlichen bildgebenden Signale ermöglicht. Dabei ist es die Aufgabe des ECCM die Läsionen aufzufinden und die Aufgabe des LSCM, die Läsionen zu charakterisieren. Durch dieses Verfahren kann die Zahl falsch-positiver Untersuchungsbefunde deutlich gesenkt werden und dem Arzt und Patienten können unnötige Folgeuntersuchungen erspart werden. ECCM und LSCM sind in ihrer erfindungsgemäßen kombinierten Verwendung Kontrastmittel mit komplementären Wirkeigenschaften.

Gegenstand der vorliegenden Erfindung ist somit ein extrazelluläres Kontrastmittel (ECCM) für die Diagnose von Läsionen in KombinationNerbindung mit einem läsionsspezifischen Kontrastmittel (LSCM). Erfindungsgemäß können die einzelnen Kontrastmittel der Kombination von ECCM und LSCM bildgebende Kontrastmittel für ein bildsynthetisches Verfahren (monomodal) oder mehrere bildsynthetische Verfahren (polymodal) sein.

Die erfindungsgemäße Kombination von ECCM und LSCM sieht eine feste zeitliche Abfolge bei der Anwendung der einzelnen Wirkstoffe vor. Im Falle des monomodalen Verfahrens wird zuerst das ECCM und dann das LCSM verabreicht. Das LSCM wird frühestens dann verabreicht, wenn der Blutspiegel des ECCM auf ein Niveau gesunken ist, welches die Detektion des LSCM ermöglicht.

Daher ist ebenfalls Gegenstand der vorliegenden Erfindung ein extrazelluläres Kontrastmittel (ECCM) für die Diagnose von Läsionen, wobei das ECCM im Falle des monomodalen Verfahrens zur Verabreichung als erstes Kontrastmittel hergerichtet ist und das LSCM zur Verabreichung nach Sinken des Blutspiegel des ECCM auf ein Niveau, das die Detektion des ECCM gestattet, als zweites Kontrastmittel hergerichtet ist. Im Falle des polymodalen Verfahrens können die Kontrastmittel so hergerichtet sein, dass beide Kontrastmittel zu einem Zeitpunkt verabreicht werden. Sie können aber im Falle des polymodalen Verfahrens so hergerichtet werden, dass das LSCM zur Verabreichung als erstes Kontrastmittel und ECCM zur Verabreichung als zweites Kontrastmittel hergerichtet ist.

Bevorzugtermaßen wird jedoch die erfindungsgemäße Kombination in der Weise angewendet, dass das ECCM zuerst verabreicht wird, um in den betreffenden zu untersuchenden Organen verdächtige Läsionen aufzuspüren. Die Aufgabe des LSCM ist es dann, durch Anreicherung in den Läsionen Informationen über die Art der Läsionen zu geben.

Im Falle des polymodalen Verfahrens können die beiden Kontrastmittel sowohl nacheinander wie im monomodalen Verfahren verabreicht werden, als auch gleichzeitig verabreicht werden.

Somit ist Gegenstand der gegenwärtigen Erfindung ein extrazelluläres Kontrastmittels (ECCM) für die Diagnose von Läsionen in Verbindung mit LSCM, wobei im Falle des polymodalen Verfahrens
- entweder das ECCM zur Verabreichung als erstes Kontrastmittel hergerichtet ist und das LSCM zur Verabreichung nach Sinken des Blutspiegel des ECCM auf ein Niveau, welches die Detektion des LSCM gestattet, als zweites Kontrastmittel hergerichtet ist oder
- das ECCM und das LSCM zur gleichzeitigen Verabreichung hergerichtet sind.

Die Kontrastmittel können auch so hergerichtet sein, dass das LSCM zur Verabreichung als erstes Kontrastmittel und ECCM zur Verabreichung als zweites Kontrastmittel hergerichtet ist. Gegenstand der vorliegenden Erfindung ist auch ein Verfahren zur Diagnose von Läsionen, wobei ein extrazelluläres Kontrastmittel (ECCM) in Kombination / Verbindung mit einem läsionsspezifischen Kontrastmittel (LSCM) verabreicht wird und bevorzugt eine unmittelbare Interpolation der bildgebenden Signale erfolgt.

Die erfindungsgemäßen ECCM sind signalgebende oder signalmodulierende Wirkstoffe für bildsynthetische Verfahren, die nach Applikation am Menschen durch die üblicherweise bolusartige Injektion schnell eine hohe Spitzenkonzentration im Blut erreichen und von diesem hohen Konzentrationsniveau im Blut durch Verteilung im Gesamtorganismus schnell wieder abfallen (alpha slope der Eliminationskinetik). Dieser Vorgang ist in der Regel innerhalb von 5 bis 10 Minuten nach Applikation abgeschlossen. Die Wirkstoffspiegel in der Zirkulation sind dann in der Regel unterhalb der bildgebenden Nachweisgrenze. Für die weitere Clearence der Wirkstoffe aus der Zirkulation ist die renale oder hepatische Eliminierung oder eine Kombination beider Prozesse entscheidend. Um schnell aus der Blutzirkulation entfernt werden zu können, dürfen die bildgebenden Wirkstoffe nur eine kleine Molekülgröße haben. Wirkstoffe mit einem Molekulargewicht von mehr als 2.000 bis 5.000 werden schon deutlich verzögert aus der Blutzirkulation entfernt im Vergleich zu Wirkstoffen mit einem Molekulargewicht von bis zu 1.000 g/mol. Daher haben die erfindungsgemäßen ECCM eine Molekülgröße von kleiner als 2000 g/mol, bevorzugt kleiner als 1000 g/mol. Eine weitere wichtige Eigenschaft ist die Hydrophilie der ECCM. Substanzen, die eine hohe Hydrophilie aufweisen werden schnell eliminiert. Substanzen, die eine geringe Hydrophilie aufweisen, werden deutlich verzögert ausgeschieden. Die Hydrophilie der erfindungsgemäßen ECCM ist durch einen Verteilungskoeffizienten von log P < -2 (kleiner minus zwei in n-Bufianof / Wasser) bevorzugt durch einen Verteilungskoeffizienten von log P < -3 (kleiner minus drei in Butanol / Wasser) charakterisiert.

Erfindungsgemäße ECCM sind Kontrastmittel für die MRT, für Röntgenstrahlenbasierte Verfahren wie z.B. die CT, für optische Verfahren, für optoakustische Verfahren, für Ultraschallverfahren und für nuklearmedizinische Verfahren. Sie sind dadurch charakterisiert, dass sie nach Injektion keine Wechselwirkungen mit Strukturen des Organismus eingehen oder durch Wechselwirkungen mit Strukturen des Organismus in ihrer signalgebenden Eigenschaft verändert werden. Ihre Bewegung wird durch die Geschwindigkeit der Verteilung und Elimination bestimmt. Nach Erreichen der Blutmaximalkonzentration ist der Vorgang der Verteilung und das Erreichen nicht mehr bildgebender Wirkstoffspiegel vorzugsweise nach 10 Minuten abgeschlossen. Daher ist die Konzentration der erfindungsgemäßen Kontrastmittel spätestens 30 Minuten nach Injektion in der Blutzirkulation soweit erniedrigt, dass das LSCM angewendet werden kann, bevorzugt spätestens nach 20 Minuten, am meisten bevorzugt spätestens nach 10 Minuten.

Besonders bevorzugt sind ECCM, bei denen der Blutspiegel mindestens nach 30 Minuten, besonders bevorzugt nach 10 bis 15 Minuten auf ein Niveau gesunken ist, das eine Verabreichung des LSCM gestattet.

Die ECCM sind Wirkstoffe, Kontrastmittel oder Effektormoleküle, wobei diese bevorzugt ausgewählt sind aus der Gruppe umfassend:
- Metallkomplexe mit paramagnetischen Metallen,
- Superparamagnetische, ferromagnetische oder ferrimagnetische Eisenoxidpartikel mit polymeren Schutzhüllen,
- Komplexgebundene, chelatorgebundene und kovalent gebundene Radionuklide
- Gasgefüllte, polymere Mikropartikel oder -vesikel
- Gasprecursor
- Organische, metallorganische oder anorganische Chromophore oder Fluorophore
- Strukturen, die biosynthetisch organische Chromophore oder Fluorophore bilden,
- Strukturen mit hohem Absorptionsquerschnitt für Röntgenstrahlen
- Strukturen mit Einfluss auf die elektrische Impedanz.

Gegenstand der Erfindung ist die Verwendung von ECCM, die paramagnetische Metallionen enthalten. Bevorzugte paramagnetische Metallionen sind Ionen der Übergangs- und Lanthanoidmetalle (z. B. Metalle der Ordnungszahlen 6-9, 21-29, 42, 43, 44, oder 57-71), insbesondere Ionen des Cr, V, Mn, Fe, Co, Ni, Cu, La, Ce, Pr, Nd, Pm, Sm, Eu, Gd, Tb, Dy, Ho, Er, Tm, Yb und Lu. Bevorzugt sind Mn, Cr, Fe, Gd und Dy. Insbesondere bevorzugt ist Gd.

Diese Ionen sind durch komplexbildende Strukturen oder Chelatorgruppen stabil gebunden oder komplexiert. Letztere sind makrozyklische oder offenkettige Polyaminocarbonsäuren. Makrozyklische Chelatorgruppen sind bevorzugt Tetraazacyclododecan-Chelatoren, wie 1,4,7,10-Tetraazacyclododecan-N,N',N",N"'-tetraessigsäure (DOTA); 1,4,7,10-Tetraazacyclododecan-N,N',N"-triessigsäure (DO3A); 1-Oxa-4,7,10-triazacyclododecan-N,N',N"-triessigsäure (OTTA); Trans(1,2)-cyclohexanodiethylentriamin pentaessigsäure (CDTPA) und Analoge von diesen, oder Ethylenaminchelatorgruppen wie N,N,N',N",N"-Diethylentriaminpentaessigsäure (DTPA), Ethylendiamintetraessigsäure (EDTA), sowie deren Derivate durch chemische Substitution and den Ethylen- und/oder Essigsäureresten. Weitere Derivate sind DTPA-BMA, DPDP, TMT und HPDO3A, Gadubotrol (Gadovist), Gadopentetsäure / Dimegluminsalz (Magnevist), Gadobensäure (Multihance), Gadodiamid (Omniscan), Gadoxelsäure / Dinatriumsalz (Primovist; Gd-EOB-DTPA), und Gadoteridol (Prohance)..

Besonders bevorzugt sind Gadubotrol (Gadovist), Gadopentetsäure / Dimegluminsalz (Magnevist), Gadobensäure (Multihance), Gadodiamid (Omniscan), Gadoxelsäure / Dinatriumsalz (Primovist; Gd-EOB-DTPA), und Gadoteridol (Prohance).

Gegenstand der Erfindung sind ECCM, die superparamagnetische Eisenoxidpartikel enthalten. Diese sind durch stabilisierende Schutzhüllen biokompatibel und verträglich. Speziell haben die Eisenoxidpartikel mit Schutzhülle einen Durchmesser von 20 - 500 nm, bevorzugt 20 - 200 nm. Schutzhüllen bestehen aus Polymeren, speziell Polysacchariden, wie Dextran. Besonders bevorzugt sind SPIOs, USPIOs, MIONs, Ferucarbutran (Resovist, Supravist).

Gegenstand der Erfindung sind ECCM, die Chromophore oder Fluorophore enthalten. Chromophore oder Fluorophore sind Strukturen, die ein ausgedehntes System delokalisierter Elektronen besitzen und innerhalb des spektralen Bereichs von 300-1400 nm absorbieren und fluoreszieren. Bevorzugt sind Chromophore oder Fluorophore mit einem Absorptions- und/oder Emissionsmaximum von 400 - 600 nm (sichtbare Fluoreszenz) sowie mit einem Absorptions- und/oder Emissionsmaximum von 650 - 1000 nm, speziell 700 - 900 nm (Nahinfrarotfluoreszenz).

Chromophore oder Fluorophore mit sichtbarer Fluoreszenz sind NBD, Fluoresceine, Rhodamine, Tetrapyrrole (z. B. Porphyrine, Protoporphyrin IX), Pyriliumfarbstaffe, Thaipyrilliumfarbstoffe, Croconiumfarbstoffe, Squariliumfarbstoffe, Benzophenoxaziniumfarbstoffe, Benzothiaphenothiaziniumfarbstoffe, Anthrachinone, Napthochinone, Phthaloylacridone, Azofarbstoffe, Diazofarbstoffe, sowie Komplexe der Lanthanoidmetalle La, Ce, Pr, Nd, Pm, Sm, Eu, Gd, Tb, Dy, Ho, Er, Tm, Yb, und Lu, Eu, Tb, Yb mit makrozyklischen oder offenkettigen Polyaminocarbonsäuren oder Polyaminocarbonsäuren-phosphorsäuren. Besonders bevorzugt ist Fluorescein.

Chromophore oder Fluorophore mit nah-infraroter Fluoreszenz sind Polymethinfarbstoffe, speziell Gyaninfarbstoffe, Merocyanine, Phthalocyanine, Naphthalocyanine, Triphenylmethine, Croconiumfarbstoffe, Squariliumfarbstoffe. Bevorzugt sind Indocyanine speziell Indocyaningrün, DODCl, DTDCI, DOTCI und DDTCI und Derivate. Besonders bevorzugt ist Indocyaningrün (ICG, CardioGreen, IC Green, DiagnoGreen).

Beispiele sind zu finden in "Topics in Applied Chemistry: Infrared absorbing dyes" Ed. M. Matsuoka, Plenum, N.Y. 1990, "Topics in Applied Chemistry: The Chemistry and Application of Dyes", Waring et al., Plenum, N.Y., 1990, "Handbook of Fluorescent Probes and Research Chemicals" Haugland, Molecular Probes Inc, 1996, DE-A-4445065, DE-A-4326466, JP-A-3/228046, Narayanan et al. J. Org. Chem. 60: 2391-2395 (1995), Lipowska et al. Heterocyclic Comm. 1: 427-430 (1995), Fabian et al. Chem. Rev. 92: 1197 (1992), WO96/23525, Strekowska et al. J. Org. Chem. 57: 4578-4580 (1992), WO (Axis) and WO96/17628.

Gegenstand der Erfindung sind auch ECCM, die aus partikulären oder vesikulären Polymeren bestehen, die Luft oder fluorinierte Gase (z. B. SF₆ oder perfluorinierte Alkane mit 1-6 C-Atomen oder andere Gase wie in WO97/29783 beschrieben) enthalten, transportieren und/oder freisetzen. Besonders geeignet sind Echovist, Levovist, Sonavist, Sonuvue und Optison.

Gegenstand der Erfindung sind ECCM, die Radionuklide enthalten. Radionuklide sind sowohl Nichtmetallnuklide als auch Metallnuklide, jeweils für die Technik SPECT (single photon emission computed tomography) oder PET (positron emission tomography).

Nichtmetallnuklide sind kovalent an Kohlenstoffe chemischer Strukturen gebunden. Ein besonders bevorzugtes Nichtmetallnuklid ist radioaktives Iod (SPECT: ¹²⁵I, ¹²³I, ¹³¹I, PET: ¹²⁴I) oder Kohlenstoff ¹¹C (PET).

Metallradionuklide sind bevorzugt ⁹⁰Y, ^{99m}Tc, ¹¹¹In, ⁴⁷Sc, ⁶⁷Ga, ⁵¹Cr, ^{177m}Sn, ⁶⁷Cu, ¹⁶⁷Tm, ⁹⁷Ru, ¹⁸⁸Re, ¹⁷⁷Lu, ¹⁹⁹Au, ²⁰³Pb und ¹⁴¹Ce (für SPECT) und ⁸⁶Y, ^{94m}Tc, ^{110m}In, ⁶⁸Ga, ⁶⁴Cu (für PET). Diese sind durch Komplexbildner oder Radiochelatoren gebunden.

Chelatoren für Metallradionuklide sind Strukturen mit Donoratomen, wie N, S, O, die in geeigneter räumlicher Anordnung die Metalle binden und einen zyklischen Metallkomplex oder Chelate bilden. Dies sind insbesondere N₃S, N₂S₂-Systeme auf Basis von Aminoalkyl, Thioalkyl, Aminocarbonyl, Thiocarbonylstrukturelementen (Kirk-Othmer Encyclopedia of Chemical Technology, Vol. 5, 339-368).

Chelatoren auf Basis von N₃S and N₂S₂ sind beispielsweise beschrieben in U.S. Patenten 4,444,690; 4,670,545; 4,673,562; 4,897,255; 4,965,392; 4,980,147; 4,988,496; 5,021,556 and 5,075,099, WO92/08494. Representative Chelatoren sind weiterhin beschrieben in U.S. Pat. No. 5,559,214 A, WO 95/26754, WO 94/08624, WO 94/09056, WO 94/29333, WO 94/08624, WO 94/08629 Al, WO 94/13327 A1 and WO 94/12216 A1; WO89/00557, U.S. Patenten 4,647,447; 5,367,080; 5,364,613. Chelate sind auch modifizierte Proteine, die z. B. ^{99m}Tc binden (U.S. Patent 5,078,985).

Chelatstrukturen sind ausgewählt aus makrozyklischen oder offenkettigen Aminocarbonsäuren. Makrozyklische Chelatorgruppen sind bevorzugt tetraazacyclododecan-Chelatoren, wie 1,4,7,10-Tetraazacyclododecan-N,N',N",N"'-Tetraessigsäure (DOTA); 1,4,7,10-tetraazacyclododecan-N,N',N"-triessigsäure (DO3A); 1-Oxa-4,7,10-triazacyclododecan-N,N',N"-triessigsäure (OTTA), sowie Dibenzo[18]krone-6, (CH₃)₆-[14]-4,11]-dien-N₄, und (2.2.2-Kryptat). Offenkettige Aminocarbonsäuren sind beispielsweise Trans(1,2)-cyclohexanodiethylentriaminpentaessigsäure (CDTPA) und Analoge von diesen, N,N,N',N",N"-Diethylentriaminpentaessigsäure (DTPA), Ethylendiamintetraessigsäure (EDTA), N-(2-hydroxy)-ethylen-diamintriessigsäure, Nitrilotriessigsäure, N,N-Di-(2-hydroxyethyl) glycin, Ethylenbis-(hydroxyphenylglycin) sowie deren Derivate durch chemische Substitution an den Ethylen- und/oder Essigsäureresten. Weitere Derivate sind DTPA-BMA, DPDP, TMT und HPD03A.

Chelatstrukturen sind darüber hinaus ausgewählt aus den Substanzklassen der Polyphosphate, wie z. B. Natriumtripolyphosphat und Hexametaphosphorsäure; 1,3-Diketone, z. B. Acetylaceton, Trifluoracetylaceton, Thenoyltrifluoraceton; Hydroxycarbonsäuren, z. B. Milchsäure, Zitronensäure, Gluconsäure, und 5-Sulfosalicylsäure; Polyamine, z. B. Ethylenediamin, Diethylenetriamin, Triethylentetraamin, Triaminotriethylamin; Aminoalkohole, z. B. Triethanolamin und N-(2-Hydroxyethyl)-ethylendiamin, aromatische heterozyklische Basen, z. B. 2,2'-Diimidazol, Picolinamin, Dipicolinamin, 1,10-Phenanthrolin; Phenole, z. B. Salicylaldehyd, Disulfopyrocatechol, Aminophenole, z. B. 8-Hydroxychinolin Oximsulfonsäure; Oxime, z. B. Dimethylglyoxim, Salicylaldoxim; Peptide mit chelatisierenden Endgruppen, z. B. Polycystein, Polyhistidin, Polyasparaginsäure, Polyglutaminsäure, Glycin-Glycin-Cystein, oder Kombinationen solcher Aminosäuren; Schiff'sche Basen, z. B. Disalicylaldehyd, 1,2-Propylendiimin; Tetrapyrrole, z. B. Porphyrine, Tetraphenylporphyrine, Benzoporphyrine, Chlorine, Tetraphenylchlorine, Benzochlorine, Bacteriochlorine, Pheophorbide; Purpurinimide, expandierte Tetra- und Pentapyrrole (Texaphyrine); Schwefelverbindungen, z. B. Toluoldithiol, Meso-2,3-dimercaptobernsteinsäure, Dimercaptopropanol, Thioglycolsäure, Natrium-diethyldithiocarbamat, Dithizon, Diethyldithiophosphorsäure, Thioharnstoff; Phosphonsäuren, z. B. Nitrilotrimethylenphosphonsäure, Ethylendiamin-tetra(methylenphosphonsäure), Hydroxyethylidendiphosphonsäure, oder Kombinationen von 2 oder mehr der genannten Strukturen.

Komplexbildner für ^{99m}Tc sind des Weiteren ^{99m}Tc(l)(H₂O)₃(CO)₃⁺, aus welchem der ^{99m}Tc-Tricarbonylkomplex mit Aminocarbonsäuren oder anderen chelatisierenden Donoratomen entsteht.

Gegenstand der Erfindung sind ECCM, die eine Absorption von Röntgenstrahlen aufweisen, speziell triiodierte Aromaten und Komplexe mit Lanthanoidionen La, Ce, Pr, Nd, Pm, Sm, Eu, Gd, Tb, Dy, Ho, Er, Tm, Yb, und Lu, Eu, Tb, Yb, bevorzugt Gd, Tb, Dy, Ho, sowie Strukturen, die triiodierte Aromaten und Komplexe mit Lanthanoidionen enthalten, sowie Iodixanol (Visipaque), Iopromid (Ultravist), loxaglinsäure (Hexabrix), Iomeprol (Imeron), lopamidol, lotrolan (Isovist), Iosurcal (Melitrast), lohexol (Omnipaque), Aminotrizoesäure (Peritrast), loxitalaminsäure-Megluminsalz (Telebrix), Iobitridol (Xenetix), und Gadolinium-DTPA

Bevorzugt sind Iodixanol (Visipaque), lopromid (Ultravist), Ioxaglinsäure (Hexabrix), Iomeprol (Imeron), Iopamidol, Iotrolan (Isovist), losurcal (Melitrast), Iohexol (Omnipaque), Aminotrizoesäure (Peritrast), loxitalaminsäure-Megluminsalz (Telebrix), lobitridol (Xenetix), und Gadolinium-DTPA

In einer besonders bevorzugten Ausführungsform ist das ECCM Gadolinium-DTPA.

Die erfindungsgemäßen läsionsspezifischen Kontrastmittel (LSCM) sind signalgebende oder signalmodulierende Wirkstoffe für bildsynthetische Verfahren, die dadurch charakterisiert sind, das sie mit Strukturen im Organismus in Wechselwirkungen treten oder durch Strukturen im Organismus in ihren signalgebenden Eigenschaften verändert werden und eine zusätzliche bildgebende Information liefern, welche die Spezifität des Verfahrens verbessern. Diese zusätzliche Information kann Information zur Anatomie, Morphologie, Funktion, Metabolismus oder molekularen Expression bestimmter Faktoren sein. Die erfindungsgemäßen Substanzen sind dadurch charakterisiert, dass sie sich nach Applikation im Läsionsgewebe kontinuierlich anreichern und im Vergleich zu ECCM in der Läsion über einen längeren Zeitraum der Untersuchung verbleiben und kein wash-in / wash-out Phänomen aufweisen. Die Anreicherung kann durch unterschiedliche Mechanismen erreicht werden, welche das Ziel verfolgen, eine schnelle Elimination aus der Blutzirkulation zu verhindern. Die erfindungsgemäßen läsionsspezifischen Wirkstoffe können an spezifische Bindungsstellen binden, sich in Zellmembranen anreichern, durch Enzymaktivität aktiviert werden, an extrazelluläre Proteine binden, durch Zellen des RES aufgenommen werden oder in die Zellen des Läsionsgewebes eindringen. Diese Substanzen sind dadurch charakterisiert, dass sie im Vergleich zu den ECCM deutlich langsamer aus der Blutzirkulation entfernt werden. Durch dieses längere Verweilen sind die Wirkstoffe in der Lage, sich in den verdächtigen Läsionen durch die genannten Mechanismen anzureichern und dort zu verweilen. Der Vorgang führt vorzugsweise nach 15 Minuten - 24 h, besonders bevorzugt nach 15 Minuten bis 3 h zu einer diagnostisch messbaren Abrgrenzung der Krankheitsläsion vom umliegenden gesunden Gewebe. Bei dieser diagnostisch messbaren Abgrenzung (Demarkation) spielt es keine Rolle, ob das LSCM sich im Gewebe der Kranhkeitsläsion oder im umliegenden gesunden Gewebe anreichert.

Bevorzugter Weise reichert sich das LSCM nach 10 Minuten in der Läsion an und verweilt dort mindestens 1 Stunde, wobei insbesondere bevorzugt die Konzentration des LSCM in der Läsion kontinuierlich im Zeitraum von 10 Minuten bis 60 Minuten ansteigt.

Erfindungsgemäße LSCM sind Kontrastmittel für die MRT, für röntgenstrahlenbasierte Verfahren wie z.B. die CT, für optische Verfahren, für optoakustische Verfahren, für Ultraschallverfahren und für nuklearmedizinische Verfahren.

Die LSCM sind Wirkstoffe, Kontrastmittel oder Effektormoleküle, wobei diese ausgewählt sind aus der folgenden Gruppe, umfassend:
- Metallkomplexe mit paramagnetischen Metallen,
- Superparamagnetische, ferromagnetische oder ferrimagnetische Eisenoxidpartikel mit polymeren Schutzhüllen,
- Komplexgebundene, chelatorgebundene und kovalent gebundene Radionuklide
- Gasgefüllte, polymere Mikropartikel oder -vesikel
- Gasprecursor
- Organische, metallorganische oder inorganische Chromophore oder Fluorophore
- Strukturen, die biosynthetisch organische Chromophore oder Fluorophore bilden,
- Strukturen mit hohem Absorptionsquerschnitt für Röntgenstrahlen
- Strukturen mit Einfluss auf die elektrische Impedanz.

Gegenstand der Erfindung ist die Verwendung von LSCM, die paramagnetische Metallionen enthalten. Bevorzugte paramagnetische Metallionen sind Ionen der Übergangs- und Lanthanoidmetalle (z. B. Metalle der Atomzahlen 6-9, 21-29, 42, 43, 44, oder 57-71), insbesondere Ionen des Cr, V, Mn, Fe, Co, Ni, Cu, La, Ce, Pr, Nd, Pm, Sm, Eu, Gd, Tb, Dy, Ho, Er, Tm, Yb und Lu. Bevorzugt sind Mn, Cr, Fe, Gd und Dy. Insbesondere bevorzugt ist Gd.

Diese Ionen sind durch komplexbildende Strukturen oder Chelatorgruppen stabil gebunden oder komplexiert. Diese sind makrozyklische oder offenkettige Polyaminocarbonsäuren. Makrozyklische Chelatorgruppen sind bevorzugt Tetraazacyclododecan-Chelatoren, wie 1,4,7,10-Tetraazacyclododecan-N,N',N",N"'-tetraessigsäure (DOTA); 1,4,7,10-Tetraazacyclodadecan-N,N',N"-triessigsäure (DO3A); 1-Oxa-4,7,10-triazacyclododecane-N,N',N"-triessigsäure (OTTA); Trans(1,2)-cyclohexanodiethylentriamin-pentaessigsäure (CDTPA) und Analoge von diesen, oder Ethylenaminchelatorgruppen wie N,N,N',N",N"-Diethylentriaminpentaessigsäure (DTPA), Ethylendiamintetraessigsäure (EDTA), sowie deren Derivate durch chemische Substitution an den Ethylen- und/oder Essigsäureresten. Weitere Derivate sind DTPA-BMA, DPDP, TMT und HPDO3A.

Besonders geeignet sind Gadofosveset (Vasovist), Gadofluorin, Gadofluorin-M, und Gadomer-17.

Gegenstand der Erfindung sind LSCM, die superparamagnetische Eisenoxidpartikel enthalten. Diese sind durch stabilisierende Die Schutzhüllen biokompatibel und verträglich. Speziell haben die Eisenoxidpartikel mit Schutzhülle einen Durchmesser von 20 - 500 nm, bevorzugt 20 - 200 nm. Schutzhüllen bestehen aus Polymeren, speziell Polysacchariden, wie Dextran.

Bevorzugt sind SPIOs, USPIOs, MIONs, Ferucarbutran (Resovist, Supravist).

Gegenstand der Erfindung sind LSCM, die Chromophore oder Fluorophore enthalten. Chromophore oder Fluorophore sind Strukturen, die ein ausgedehntes System delokalisierter Elektronen besitzen und innerhalb des spektralen Bereichs von 300-1400 nm absorbieren und fluoreszieren. Bevorzugt sind Chromophore oder Fluorophore mit einem Absorptions- und/oder Emissionsmaximum von 400 - 600 nm (sichtbare Fluoreszenz) sowie mit einem Absorptions- und/oder Emissionsmaximum von 650-1000 nm, speziell 700-900 nm (Nahinfrarotfluoreszenz).

Chromophore oder Fluorophore mit sichtbarer Fluoreszenz sind NBD, Fluoresceine, Rhodamine, Tetrapyrrole (z. B. Porphyrine, Protoporphyrin IX), Pyriliumfarbstoffe, Thaipyrilliumfarbstoffe, Croconiumfarbstoffe, Squariliumfarbstoffe, Benzophenoxaziniumfarbstoffe, Benzothiaphenothiaziniumfarbstoffe, Anthrachinone, Napthochinones, Phthaloylacridone, Azofarbstoffe, Diazofarbstoffe, sowie Komplexe der Lanthanidmetalle La, Ce, Pr, Nd, Pm, Sm, Eu, Gd, Tb, Dy, Ho, Er, Tm, Yb, and Lu Eu, Tb, Yb mit makrozyklischen oder offenkettigen Polyaminocarbonsäuren oder Polyaminocarbonsäuren-phosphorsäuren. Besonders bevorzugt ist Fluorescein.

Chromophore oder Fluorophore mit nah-infraroter Fluoreszenz sind Polymethinfarbstoffe, speziell Cyaninfarbstoffe, Merocyanine, Phthalocyanine, Naphthalocyanine, Triphenylmethine, Croconiumfarbstoffe, Squariliumfarbstoffe. Bevorzugt sind Indocyanine speziell Indocyaningrün, DODCI, DTDCI, DOTCI und DDTCI und Derivate. Besonders bevorzugt ist SF64 (Omocianine).

Beispiele sind zu finden in "Topics in Applied Chemistry: Infrared absorbing dyes" Ed. M. Matsuoka, Plenum, N.Y. 1990, "Topics in Applied Chemistry: The Chemistry and Application of Dyes", Waring et al., Plenum, N.Y., 1990, "Handbook of Fluorescent Probes and Research Chemicals" Haugland, Molecular Probes Inc, 1996, DE-A-4445065, DE-A-4326466, JP-A-3/228046, Narayanan et al. J. Org. Chem. 60: 2391-2395 (1995), Lipowska et al. Heterocyclic Comm. 1: 427-430 (1995), Fabian et al. Chem. Rev. 92: 1197 (1992), WO96/23525, Strekowska et al. J. Org. Chem. 57: 4578-4580 (1992), WO (Axis) and WO96/17628.

Gegenstand der Erfindung ist die Verwendung von Wirkstoffen, die nach Verabreichung der Wirkstoffe Chromophore oder Fluorophore biosynthetisch bilden. Bevorzugt genannt ist 5-Aminolaevulinsäure (5-ALA) und Ester von 5-ALA.

Gegenstand der Erfindung sind LSCM, die aus partikulären oder vesikulären Polymeren bestehen, die Luft oder fluorinierte Gase (z. B. SF6 oder perfluorinierte Alkane mit 1-6 C-Atom oder andere Gase wie in WO97/29783 beschrieben) enthalten, transportieren und/oder freisetzen. Besonders bevorzugt sind partikuläre Polymere, die an zielsuchende Peptide oder Proteine gekoppelt sind.

Gegenstand der Erfindung sind LSCM, die Radionuklide enthalten. Radionuklide sind sowohl Nichtmetallnuklide als auch Metalinuklide, jeweils für die Technik SPECT (single photon emission computed tomography) oder PET (positron emission tomography).

Nichtmetallnuklide sind kovalent an Kohlenstoffe chemischer Strukturen gebunden. Ein besonders bevorzugtes Nichtmetallnuklid ist radioaktives Iod (SPECT: ¹²⁵I, ¹²³I, ¹³¹I, PET: ¹²⁴I) oder Kohlenstoff ¹¹C (PET).

Metallradionuklide sind bevorzugt ⁹⁰Y, ^{99m}Tc, ¹¹¹In, ⁴⁷Sc, ⁶⁷Ga, ⁵¹Cr, ^{177m}Sn, ⁶⁷Cu, ¹⁶⁷Tm, ⁹⁷Ru, ¹⁸⁸Re, ¹⁷⁷Lu, ¹⁹⁹Au, ²⁰³Pb und ¹⁴¹Ce (für SPECT) und ⁸⁶Y, ^{94m}Tc, ^{110m}In, ⁶⁸Ga, ⁶⁴Cu (für PET). Diese sind durch Komplexbildner oder Radiochelatoren gebunden.

Chelatoren für Metallradionuklide sind Strukturen mit Donoratomen, wie N, S, O, die in geeigneter räumlicher Anordnung die Metalle binden und einen zyklischen Metallkomplex oder Chelate bilden. Dies sind insbesondere N₃S, N₂S₂-Systeme auf Basis von Aminoalkyl, Thioalkyl, Aminocarbonyl, Thiocarbonylstrukturelementen (Kirk-Othmer Encyclopedia of Chemical Technology, Vol. 5, 339-368).

Chelatoren auf Basis von N₃S and N₂S₂ sind beispielsweise beschrieben in U.S. Patenten 4,444,690; 4,670,545; 4,673,562; 4,897,255; 4,965,392; 4,980,147; 4,988,496; 5,021,556 and 5,075,099, WO92/08494. Repräsentative Chelatoren sind weiterhin beschrieben in U.S. Pat. No. 5,559,214 A, WO 95/26754, WO 94/08624, WO 94/09056, WO 94/29333, WO 94/08624, WO 94/08629 Al, WO 94/13327 A1 and WO 94/12216 A1; WO89/00557, U.S. Patenten 4,647,447; 5,367,080; 5,364,613. Chelate sind auch modifizierte Proteine, die z. B. ^{99m}Tc binden (U.S. Patent 5,078,985).

Cheiatstrukturen sind ausgewählt aus makrozyklische oder offenkettige Aminocarbonsäuren. Makrozyklische Chelatorgruppen sind bevorzugt Tetraazacyclododecan-Chelatoren, wie 1,4,7,10-Tetraazacyclododecan-N,N',N",N"'-tetraessigsäure (DOTA); 1,4,7,10-Tetraazacyclododecan-N,N',N"-triessigsäure (DO3A); 1-Oxa-4,7,10-triazacyclodadecan-N,N',N"-triacetic acid (OTTA), sowie Dibenzo[18]krone-6, (CH₃)₆-[14]-4,11]-dien-N₄, und (2.2.2-Kryptat). Offenkettige Aminocarbonsäuren sind beispielsweise Trans(1,2)-cyclohexanodiethylentriaminpentaessigsäure (CDTPA) und Analoge von diesen, N,N,N',N",N"-Diethylentriaminpentaessigsäure (DTPA), Ethylendiamintetraessigsäure (EDTA), N-(2-hydroxy)-ethylen-diamintriessigsäure, Nitrilotriessigsäure, N,N-Di-(2-hydroxyethyl)-glycin, Ethylenbis-(hydroxyphenylglycin) sowie deren Derivate durch chemische Substitution an den Ethylen- und/oder Essigsäureresten. Weitere Derivate sind DTPA-BMA, DPDP, TMT und HPD03A.

Chelatstrukturen sind darüber hinaus ausgewählt aus den Substanzklassen der Polyphosphate, wie z. B. Natriumtripolyphosphat und Hexametaphosphorsäure; 1,3-Diketone, z. B. Acetylaceton, Trifluoracetylaceton, Thenoyltrifluoraceton; Hydroxycarbonsäuren, z. B. Milchsäure, Zitronensäure, Gluconsäure, und 5-Sulfosalicylsäure; Polyamine, z. B. Ethylenediamin, Diethylentriamin, Triethylentetraamin, Triaminotriethylamin; Aminoalkohole, z. B. Triethanolamin und N-(2-Hydroxyethyl)-ethylendiamin; aromatische heterozyklische Basen, z. B. 2,2'-Diimidazol, Picolinamin, Dipicolinamin, 1,10-Phenanthrolin; Phenole, z. B. Salicylaldehyd, Disulfopyrocatechol; Aminophenole, z. B. 8-Hydroxychinolin Oximsulfonsäure; Oxime, z. B. Dimethylglyoxim, Salicylaldoxim; Peptide mit chelatisierenden Endgruppen, z. B. Polycystein, Polyhistidin, Polyasparaginsäure, Polyglutaminsäure, Glycin-Glycin-Cystein, oder Kombinationen solcher Aminosäuren; Schiff'sche Basen, z. B. Disalicylaldehyd, 1,2-Propylendiimin; Tetrapyrrole, z. B. Porphyrine, Tetraphenylporphyrine, Benzoporphyrine, Chlorine, Tetraphenylchlorine, Benzochlorine, Bacteriochlorine, Pheophorbide; Purpurinimide, expandierte Tetra- und Pentapyrrole (Texaphyrine); Schwefelverbindungen, z. B. Toluendithiol, Meso-2,3-dimercaptobernsteinsäure, Dimercaptopropanol, Thioglycolsäure, Natrium diethyldithiocarbamat, Dithizon, Diethyldithiophosphorsäure, Thioharnstoff; Phosphonsäuren, z. B. Nitrilotrimethylenphosphonsäure, Ethylendiamin-tetra(methylenphosphonsäure), Hydroxyethylidendiphosphonsäure, oder Kombinationen von 2 oder mehr der genannten Strukturen.

Komplexbildner für ^{99m}Tc sind des weiteren ^{99m}Tc(l)(H₂O)₃(CO)₃⁺, aus welchem der ^{99m}Tc-Tricarbonylkomplex mit Aminocarbonsäuren oder anderen chelatisierenden Donoratomen entsteht.

Chelatorstrukturen und Komplexbildner sind über Linker an Träger-, Vektor-, Targeting- oder Transportmoleküle, z. B. Polymere, Proteine, Peptide, Antikörper, Oligonukleotide, Polysaccharide und deren Kombinationen und Derivate gebunden. Chelatorstrukturen und Komplexbildner sind dabei verbunden über funktionelle Gruppen des Chelat-Rückgrats, über Derivatisierung von Donorgruppen zu derivatisierten Donorgruppen, z. B. Säuren zu Amiden, Alkoholen zu Ethern, Thiolen zu Thioethern, oder über freie Koordinationsstellen des Metalls. Die Kopplung an Träger-, Vektor-, Targeting- oder Transportmoleküle kann in molaren Verhältnissen von 1 bis 100 erfolgen. Linker und Derivate für Chelatoren sind in WO94/08629, WO94/09056, WO96/20754 beschrieben.

Bevorzugt sind die Strukturen ^{99m}Tc-Medronat, ^{99m}Tc -Sestamibi, ^{99m}Tc-ECD, ^{99m}Tc-MAG3, ¹¹¹In-DTPA-octreotid, ¹¹¹In-DTPA-octreotat, ¹⁸F-Fluordesoxyglucose (FDG), ¹⁸F-Dopamin, ¹⁸F-L-DOPA, ¹⁸F-Fluorcholin, ¹⁸F-Fluormethylethylcholin, ¹⁸F-Fluordihydrotestosteron, ⁶⁸Ga-NODAGATOC, ⁶⁸Ga-DOTATOC.

Gegenstand der Erfindung sind LSCM, die eine Absorption von Röntgenstrahlen aufweisen, speziell triiodierte Aromaten und Komplexe mit Lanthanoidionen La, Ce, Pr, Nd, Pm, Sm, Eu, Gd, Tb, Dy, Ho, Er, Tm, Yb, und Lu, Eu, Tb, Yb, bevorzugt Gd, Tb, Dy, Ho, sowie Strukturen, die triiodierte Aromaten und Komplexe mit Lanthanoidionen enthalten.

Ein besonderer Vorteil der erfindungsgemäßen Kombination von ECCM und LSCM ist die Möglichkeit, hochauflösende bildgebende Verfahren einzusetzen. Bei der alleinigen Verwendung von ECCM war der Einsatz hochauflösender bildgebender Verfahren nicht möglich, da die zur Verfügung stehenden Messintervalle sehr kurz sind. Die erfindungsgemäße Kombination von ECCM mit LSCM ermöglicht jedoch den Einsatz hochauflösender bildsynthetischer Verfahren, um spezifische morphologische Information zu erhalten.

Die Beurteilung genauer morphologischer Details ist bei allen bildsynthetischen Verfahren an eine hohe räumliche Auflösung gebunden. Für die Akquisition solcher hochauflösender Aufnahmen ist jedoch eine längere Untersuchungszeit notwendig. Darüber hinaus müssen Messverfahren angewendet werden, die für die bildsynthetische Darstellung der ECCM ungeeignet sind, da eine Erhöhung der räumlichen Auflösung zu einer deutlichen Verlängerung der Messzeit und einer Reduktion des Signal-zu-Rausch-Verhältnisses führt. Darüber hinaus sind hochauflösende Messverfahren ungeeignet, schnelle Veränderungen der Kontrastmittelverteilung, wie sie für die schnelle Verteiluung von ECCM notwendig sind, zu erfassen.

Die einzelnen Komponenten der erfindungsgemäßen Kombination von ECCM und LSCM können bildgebende Kontrastmittel oder signalgebende Wirkstoffe für ein bildsynthetisches Verfahren (monomodal) oder mehrere (polymodal, multimodal) bildsynthetische Verfahren sein.

Die monomodalen Verfahren können ausgewählt sein aus der Gruppe umfassend: MRT, PET, CT, Optical Imaging, Ultraschall, SPECT, X-Ray.

Bildsynthetische Verfahren, die geeignet sind, polymodale Kombinationen von ECCM und LSCM darzustellen, sind Fusionsverfahren unterschiedlicher bildgebender Verfahren, wie PET-CT, PET-MRT, PET-Optical Imaging, MRT-CT, Ultraschall-Optical Imaging, PET-SPECT, SPECT-CT, MRT-Optical Imaging und SPECT-MRT.

Bei der Anwendung einer erfindungsgemäßen Kombination, die mittels polymodaler Verfahren dargestellt wird, werden Vorrichtungen und Software eingesetzt, welche die Signale der einzelnen Komponenten örtlich und zeitlich bildlich getrennt darstellt und für jede verdächtige Läsion oder verdächtigen Bereich einen automatischen Vergleich der örtlichen und zeitlichen Signalintensitäten vornimmt.

Bei der monomodalen Anwendung der erfindungsgemäßen Kombination wird das ECCM beispielsweise zuerst appliziert und in einem zeitlichen Abstand folgend dann das LSCM. Das Zeitintervall zwischen der Applikation des ECCM und LSCM ist so bemessen, dass die Applikation des LSCM erst erfolgt, wenn der Blutspiegel des ECCM auf ein niedriges Niveau zurückgekehrt ist und nicht mehr mit der nachfolgenden Applikation des LSCM interferiert. Dadurch können sich die Signale der bildgebenden Wirkstoffe nicht überlappen und gegenseitig beeinflussen. Da das ECCM erst ca. 10 Minuten nach Applikation auf ein solches niedriges Niveau zurückgefallen ist, muss die nachfolgende Applikation zeitlich versetzt durchgeführt werden.

Wird die erfindungsgemäße Kombination von ECCM und LSCM mittels polymodaler bildsynthetischer Verfahren dargestellt, können das ECCM und das LSCM auch zeitgleich oder mit nur sehr geringem zeitlichem Versatz appliziert werden. Durch die Verwendung unterschiedlicher bildsynthetischer Verfahren wird eine Überlappung der einzelnen Signalkomponenten vermieden. Die Signalkomponenten des ECCM und des LSCM können getrennt dargestellt werden. Die nahezu zeitgleiche Applikation ist vor allem dann angezeigt, wenn Radioisotope mit kurzer Verfallszeit verwendet werden. Dies ist z. B der Fall, wenn ¹⁸F-basierte PET Tracer mit dem MRT ECCM Gd-DTPA kombiniert werden.

Es ist also die Kombination aus ECCM und LSCM Gegenstand der vorliegenden Erfindung in der Verwendung als:
- monomodal zeitlich versetzte Applikation, z. B. monomodale Messung in 0 - 15 Minuten nach Injektion des ECCM und zweite Messung 15 Minuten bis 24 h der Injektion des LSCM
- polymodal zeitlich versetzte Applikation
- polymodal zeitgleiche Applikation

Die zeitlich versetzte Applikation der einzelnen Kombinationskomponenten kann durch verschiedene Vorrichtungen erreicht werden. Die erfindungsgemäße Kombination kann durch Zwei- oder Mehrkammerspritzen oder -kartuschen vorgenommen werden. Eine weitere Vorrichtung zur Darreichung der erfindungsgemäßen Kombination sind Vorrichtungen, welche die zeitlich gesteuerte Applikation der einzelnen Komponenten vornehmen.

Daher ist ebenfalls Gegenstand der vorliegenden Erfindung eine Applikationsvorrichtung zur kombinierten Applikation eines extrazellulären Kontrastmittels (ECCM) für die Diagnose von Läsionen in Kombination/Verbindung mit einem läsionsspezifischen Kontrastmittel (LSCM), wobei diese Applikationsvorrichtung mindestens zwei Kammern oder Aufnahmebehälter für die getrennte Aufnahme und Applikation des ECCM und des LSCM aufweist.

Bei der genannten Appükationsvorrichtung kann es sich um eine Zwei- oder Mehrkammerspritze handeln. Es kann sich auch um eine Zwei- oder Mehrkammerkartusche handeln.

### Beispiel 1:

Ein Patient mittleren Alters. Dieser Patient war an einem bösartigen Hirntumor, einem Glioblastom, erkrankt und behandelt worden. Die Behandlung umfasste neben einer Operation auch eine Strahlentherapie des Gehirns mit erhöhter Strahlendosis im ehemaligen Operationsgebiet unter Einsatz gezielter stereotaktischer Techniken. Nach ca. einem halben Jahr verschlechtert sich die anfänglich gute klinische Situation des Patienten wieder. Die klinische Untersuchung ergibt den Verdacht auf ein erneutes Tumorwachstum.
Die diagnostische Bildgebung mittels einer PET-CT Gerätekombination kann sowohl Tumore mit Hilfe von Röntgenkontrastmitteln als auch mit Hilfe von PET-Isotopen darstellen. Mit einem extrazellulären CT-Kontrastmittel wie z.B. Ultravist® zeigt sich eine inhomogen Kontrastmittel anreichernde Region im Bereich des ehemaligen Tumorbettes. Differentialdiagnostisch kommt hier neben einem erneuten Auftreten eines Tumors, einem Lokalrezidiv, ein durch die hohe Bestrahlung bedingter Zelluntergang, eine Radionekrose, in Frage.

Das zeitgleich injizierte PET-Isotop ¹⁸F - Fluordeoxyglukose (FDG) als LSCM klärt die Differentialdiagnose: die fehlende Anreicherung des LSCM in den Zellen der Region, die in der CT eine Anreicherung mit ECCM gezeigt hatten, beweist das Vorliegen einer Radionekrose. Das weitere therapeutische Vorgehen besteht in der Gabe von Corticoiden, die Prognose des Patienten ist deutlich besser als wenn ein Lokalrezidiv vorgelegen hätte.

### Beispiel 2:

Ein Patient, typischerweise zwischen 50 und 70 Jahren alt, wird wegen Blutablagerungen auf dem Stuhlgang zur weiteren Diagnostik und Therapie in die Klinik eingeliefert. Die durchgeführte Dickdarmspiegelung ergab dabei einen bösartigen Tumor des Darmes. In der ebenfalls durchgeführten Ultraschalluntersuchung der Leber fand sich ein einzelner, umschriebener Herd im rechten Leberlappen, der den Verdacht auf eine Tochtergeschwulst in der Leber, eine Lebermetastase, ergab.
Die durchgeführte Magnetresonanztomographie zuerst mit einem extrazellulären Kontrastmittel (ECCM, z. B. Magnevist®) und direkt anschließend mit einem läsionsspezifischen Kontrastmittel (LSCM, z. B. Resovist®) bestätigte im ersten Schritt das Vorhandensein eines Leberherdes im rechten Leberlappen durch das ECCM. Eine Differenzierung der Art des Tumors gelingt hierbei jedoch nicht. Erst die Anreicherung des Tumors mit dem LSCM zeigte in diesem Fall, dass es sich bei den Zellen dieser Raumforderung um Leberzellen und nicht um Tumorzellen handeln muss. Eine Tochtergeschwulst des Darmtumors konnte somit ausgeschlossen, die Diagnose eines gutartigen Blutschwammes der Leber durch die kombinierte Kontrastmitteluntersuchung gesichert werden. Der Patient wird einer normalen Tumoroperation des Darmes zugeführt.

## Patentansprüche

1. Extrazelluläres Kontrastmittel (ECCM) für die Diagnose von Läsionen in KombinationNerbindung mit einem läsionsspezifischen Kontrastmittel (LSCM).

2. Extrazelluläres Kontrastmittel (ECCM) für die Diagnose von Läsionen gemäß Anspruch 1, wobei die einzelnen Kontrastmittel der Kombination von ECCM und LSCM bildgebende Kontrastmittel für ein bildsynthetisches Verfahren (monomodal) oder mehrere bildsynthetische Verfahren (polymodal) sind.

3. Extrazelluläres Kontrastmittel (ECCM) für die Diagnose von Läsionen gemäß Anspruch 1 oder 2, wobei im Falle des monomodalen Verfahrens das ECCM und das LSCM nacheinander zu verabreichen ist, wobei sowohl das ECCM als auch das LSCM zuerst verabreicht werden kann.

4. Extrazelluläres Kontrastmittel (ECCM) für die Diagnose von Läsionen gemäß Anspruch 1 oder 2, wobei im Falle des polymodalen Verfahrens
a. entweder das ECCM und das LSCM nacheinander zu verabreichen sind oder
b. das ECCM und das LSCM gleichzeitig zu verabreichen sind.

5. Extrazelluläres Kontrastmittel (ECCM) für die Diagnose von Läsionen in Kombination/Verbindung mit einem läsionsspezifischen Kontrastmittel (LSCM) gemäß einem der Ansprüche 1 - 4, wobei das ECCM und das LSCM mit einem zeitlichen Abstand von mindestens 10 bis 15 Minuten zu verabreichen sind.

6. Extrazelluläres Kontrastmittel (ECCM) für die Diagnose von Läsionen in Kombination/Verbindung mit einem läsionsspezifischen Kontrastmittel (LSCM) gemäß einem der Ansprüche 1 - 5, wobei das ECCM Gadolinium-DTPA ist.

7. Extrazelluläres Kontrastmittel (ECCM) für die Diagnose von Läsionen in KombinationNerbindung mit einem läsionsspezifischen Kontrastmittel (LSCM) gemäß einem der Ansprüche 1 - 6, wobei sich das LSCM nach 10 Minuten in der Läsion angereichert hat und mindestens 1 Stunde dort verweilt.

8. Extrazelluläres Kontrastmittel (ECCM) für die Diagnose von Läsionen in KombinationNerbindung mit einem läsionsspezifischen Kontrastmittel (LSCM) gemäß einem der Ansprüche 1 - 7, wobei eine unmittelbare Interpolation der bildgebenden Signale erfolgt.

9. Applikationsvorrichtung zur kombinierten Applikation eines extrazellulären Kontrastmittels (ECCM) für die Diagnose von Läsionen in KombinationNerbindung mit einem läsionsspezifischen Kontrastmittel (LSCM), wobei diese Applikationsvorrichtung die getrennte Aufnahme und Applikation des ECCM und des LSCM ermöglicht.

10. Applikationsvorrichtung gemäß Anspruch 9, wobei diese Applikationsvorrichtung mindestens zwei Kammern oder Aufnahmebehälter für die getrennte Aufnahme und Applikation des ECCM und des LSCM aufweist.

11. Verfahren zur Diagnose von Läsionen, wobei ein extrazelluläres Kontrastmittel (ECCM) in Kombination / Verbindung mit einem läsionsspezifschen Kontrastmittel (LSCM) verabreicht wird und bevorzugt eine unmittelbare Interpolation der bildgebenden Signale erfolgt.
